# EUROPEAN PATENT SPECIFICATION

(11) **EP 3 256 046 B1**
(45) Date of publication and mention of the grant of the patent: **30.10.2019**
(21) Application number: 16711691.2
(22) Date of filing: 10.02.2016
(51) Int. Cl.: A61B 5/1172, C05F 11/02

(54) **USE OF HUMIC ACID BASED POWDERS FOR LATENT FINGERPRINT DETECTION**
VERWENDUNG VON HUMINSÄUREBASIERTE PULVER ZUR DETEKTION LATENTER FINGERABDRÜCKE
UTILISATION DE POUDRES À BASE D'ACIDE HUMIQUE POUR DÉTECTION D'EMPREINTES DIGITALES LATENTES

(30) Priority: 10.02.2015 TR 201501577
(43) Date of publication of application: 20.12.2017
(73) Proprietor: Tubitak, 06100 Ankara (TR)
(72) Inventor: ÇELIKEL, Tugba, 41470 Gebze, Kocaeli (TR); GÜRTEKIN SEDEN, Merve, 41470 Gebze, Kocaeli (TR); DOGAN, Hacer, 41470 Gebze, Kocaeli (TR); EROL, Melek, 41470 Gebze, Kocaeli (TR)
(86) International application number: PCT/IB2016/050693
(87) International publication number: WO 2016/128903

(56) References cited:
- WO-A1-2005/066632
- CN-A- 102 746 845
- US-A- 1 259 981
- US-A- 4 176 205
- US-A1- 2010 310 755
- US-B1- 6 299 674
- Henry C. Lee and R.E. Gaensslen: "Advances in Fingerprint Technology - second edition", 15 June 2001 (2001-06-15), CRC Press, XP002757527, ISBN: 0849309239 page 108 - page 112

## Description

### Technical Field of the Invention

Fingerprints in crime scenes are divided into two parts as visible and latent. Colored contaminants (blood, fat, ink etc.) on the suspect's hand make fingerprints visible. However, in the case of that the color contaminants don't present on skin, most of the fingerprints in crime scenes are latent and in order to make them visible, markers such as spray or powder forms are used. This invention relates to the production and the application of fingerprint powders, which have been used to make visible the fingerprints and have been the most common method about the catching criminals at the crime scene investigations, containing humic acid or its salts (humates) which are eco-friendly and alternative to common powders.

### Background of the Invention

Biometric marks form an important step of the crime scene investigation process. Various markers are used to make visible the latent fingerprints at crime scene investigation. To obtain an effective marker used for the detection of fingerprint, it should make at least one physical or chemical bond with fatty acids, amino acids or one of the other compounds found on the fingerprints, but it should not make bond with the surface containing fingerprints. Fingerprint markers are generally in the form of powder or spray. Fingerprint powders help for the detection of suspect's fingerprints clinging to the aqueous component like perspiration in fresh fingerprints and clinging of sebaceous liquid to the oily components in old fingerprints. Electrostatic attraction between powder particles and fingerprint supports that clinging. Quality of the powder adhesion to fingerprint depends upon the size and shape of the powder particles. Fingerprint powders are applied by using fingerprint brushes to the fingerprint which was left on the surfaces. Efficiency and effectiveness of the dusting depend on the chemical and physical properties of powder, brush type, attention shown during dusting, and the applicant's expertness. The detected fingerprints using powders are taken to the crime scene investigation branches by transferring with fingerprint foils or by photographing.

Humic acid is obtained by precipitating of water-soluble alkali humates extracted with alkaline solutions from coal such as lignite or Leonardite with acid. Until today, there have been many studies and publications about characteristics of the fingerprints and the methods of making traces visible, however any work in the literature related to the use of humic acid and its salts in the field of fingerprint detection have not been found. In the patent numbered as US1259981, a study of the detection and stabilization of latent fingerprint by dusting was disclosed, in US1497971, the method related to the application of the powder form resins, which were mixed with white colored or colorant materials, to the surface and subsequently adhering it to the surface via heating was developed for the identification of latent fingerprints on paper or other surfaces, a method related to various fingerprint powders and detection of fingerprints was explained in US4176205. In the present invention, fingerprint powders containing various humic acids have been produced and used to detect the latent fingerprints.

Humic acid salts were obtained as the result of alkali (NaOH, KOH, etc.) extraction of lignite or Leonardite based brown coals. Divalent (Ca, Mg, Ba) and trivalent (Fe, Al) salts were obtained from monovalent Na and K salts of humic acid. In the present invention; potassium, sodium, barium, magnesium, calcium, aluminum, iron salts of humic acid and pure humic acid were used as fingerprint powder. Since the fingerprints are detected as light brown color, it was foreseen that the fingerprint should be darker in order to form contrast with the surface and, black fingerprint powders were produced and used in this invention by mixing humic acid based fingerprint powders with carbon black .

### Brief Description of the Invention

Fingerprint powders used at present contain materials such as iron oxide, carbon black, aluminum. In the invention, humic acid and its various salts (humate), which have been used even in areas such as food and pharmaceutical and which were more economical, were used. In the invention, it was aimed the production of fingerprint powders containing humic acid or its various salts by grinding and mixing and the application of them. In this case, the amount of carbon black needed to be used in the black fingerprint powder was reduced in the rate of 80-88%. Economical contribution will be provided with the use of humic acid and its salts produced from coals such as lignite and Leonardite.

### Brief Description of the Drawings

Figure 1- Comparative table for detection performances of humic acid fingerprint powder and carbon doped humic acid fingerprint powders on detecting fingerprints left and aged for 1 day on glass, PVC and metal surfaces is given.
Figure 2- Comparative table for detection performances of Na-humate and K- humate fingerprint powder (+1 valent humates) and carbon doped fingerprint powder left on the glass, PVC and metal surfaces and then 1 day aged is given.
Figure 3- Comparative table for detection performances of Ca-humate, Mg-humate and Ba-humate fingerprint powder (+2 valent humates) and carbon doped fingerprint powder left on the glass, PVC and metal surfaces and then 1 day aged is given.
Figure 4- Comparative table for detection performances of Fe-humate and Al-humate fingerprint powder (+3 valent humates) and carbon doped fingerprint powder left on the glass, PVC and metal surfaces and then 1 day aged is given.

### Detailed Description of the Invention

### Application of Humic Acid and its Salts Directly On Latent Fingerprints

Water-soluble sodium and potassium salts of humic acid are obtained as the result of extraction with various alkalis (KOH, NaOH, etc.) from Lignite or Leonardite based brown coals. Calcium, barium, magnesium, iron and aluminum humates are obtained with the ion-exchange reaction of Na-Humate with various metal salts (CaCl₂, CaCl₂, FeCl₃, etc.). In the present invention, the potassium, sodium, barium, magnesium, calcium, aluminum, iron salts of humic acid and pure humic acid were used as fingerprint powder. Fingerprint powders prepared in the present invention are able to determine traces which aged at various times.

Potassium, sodium, barium, magnesium, calcium, aluminum, iron salts of humic acid and pure humic acid were grinded separately by using ball mill (Fritsch Planetary Mono Mill Pulverisette) to obtain the particle size of 10-20 µm, since the particle size is an important factor for a high quality performance of trace detection in fingerprint powder. According to this, particle sizes of humic acid and its salts were obtained between 10-20 µm as the result of grinding operation at 400-600 rpm speed for 40-120 minutes by using appropriate number (40-50 pieces) of grinding balls with appropriate diameter (10 mm).

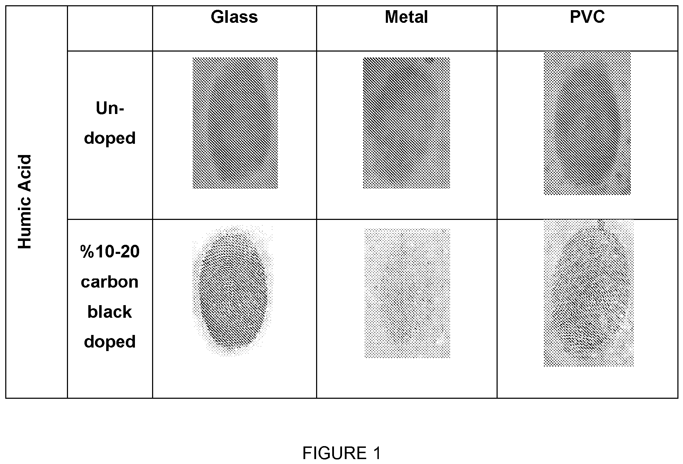

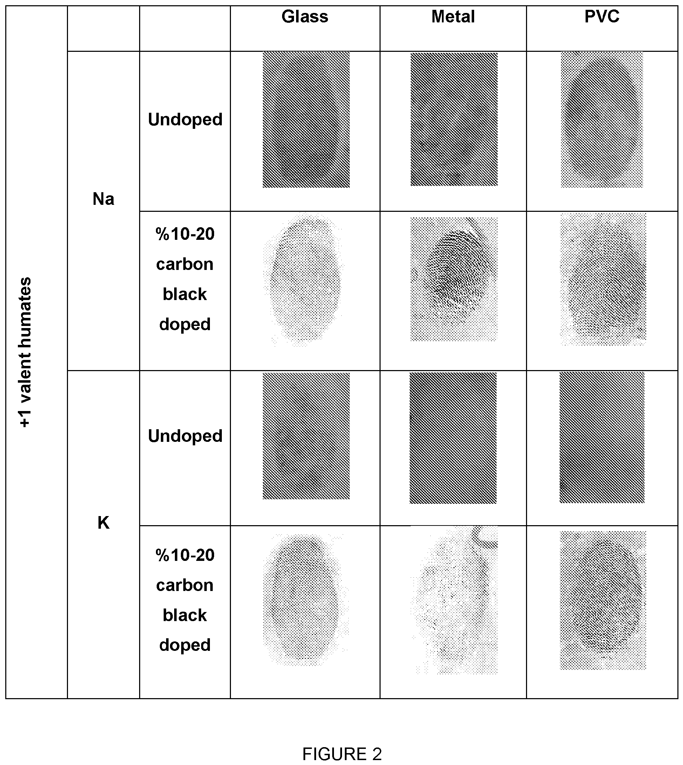

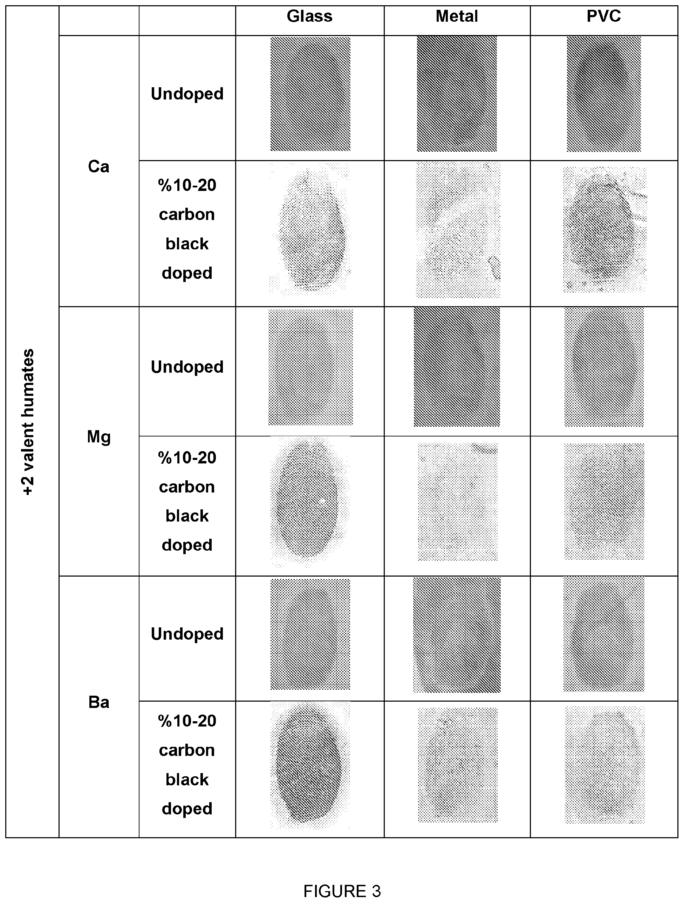

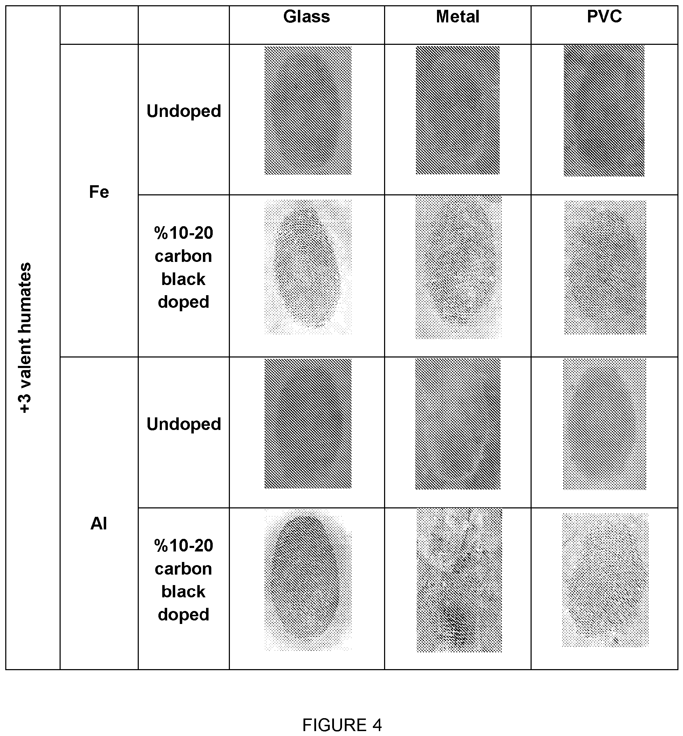

Fine brushes, which are made of glass fiber or fine animal hair and do not scratch the traces, and fingerprint powders are used for detection of the latent fingerprints at crime scenes. The application is made by making touch without pressing the brush to fingerprint powder in the powder container and rotating without pressing it on the smooth surface on which have fingerprints. The fingerprints which are left on glass, PVC and metal surfaces and then 1 day aged were detected by dusting with the produced humic acid and humate based powders and comparative tables are given in Figure 1, Figure 2, Figure 3 and Figure 4.

### Doping Humic Acid and its Salts with Carbon Black and Application to Latent Fingerprints

There should be a color contrast between the surfaces and the used fingerprint agent for detection of fingerprints at crime scenes. Although the fingerprints detected with powders containing pure humic acid and its salts have adequate qualifications, the color was obtained as light brown; therefore carbon black which is a black colorant agent was added to improve the contrast on light-colored surfaces. Carbon black was added to each type of humic acid and its salts in the ratio of 10-20% and fingerprint powder was prepared by mixing at 400 rpm speed for 20 minutes in ball mill (Fritsch Pulverisette Mill Planetary Mono) without using balls. The preparation conditions of carbon black doped humic acids and its salts are given in Table 1.

The fingerprints which are left on glass, PVC and metal surfaces and then 1 day aged were detected by dusting with the produced doped humic acid and humate based powders and comparative tables are given in Figure 1, Figure 2, Figure 3 and Figure 4.

**Table 1. Preparation Conditions of Doped Humic Acid and Humate Fingerprint Powders**

| **Sample Composition (wt. %)** | **Mixing time** |
|---|---|
| 80-90 % Humic Acid + 10-20% carbon black | 20 min |
| 80-90 % Al-humate + 10-20% carbon black | 20 min |
| 80-90 %Ba-humate + 10-20 % carbon black | 20 min |
| 80-90% Mg-humate + 10-20 % carbon black | 20 min |
| 80-90% Fe-humate + 10-20 % carbon black | 20 min |
| 80-90 % Ca-humate + 10-20 % carbon black | 20 min |
| 80-90% K-humate + 10-20 % carbon black | 20 min |
| 80-90% Na-humate + 10-20 % carbon black | 20 min |

## Claims

1. Use of a powder comprising humic acid or humic acid salts (humate) for fingerprint detection.

2. Use of the powder according to claim 1, wherein said powder further comprises about 10 -20% carbon black by weight.

3. Use of the powder according to claim 1, wherein said powder comprises the humic acid salts in the +1 valent of either Na or K salt.

4. Use of the powder according to claim 1, wherein said powder comprises the humic acid salts in the +2 valent of either Ca, Mg or Ba salt.

5. Use of the powder according to claim 1, wherein said powder comprises the humic acid salts in the +3 valent of either Fe or Al salt.

6. Use of the powder according to claim 1, wherein said powder has the average grain size between 10 - 20 µm.

## Patentansprüche

1. Verwendung eines Pulvers zur Fingerabdruckerkennung, das Huminsäure oder Huminsäuresalze (Humat) enthält.

2. Verwendung des Pulvers nach Anspruch 1, worin das Pulver weiterhin etwa 10 bis 20% seines Gewichtes Ruß enthält.

3. Verwendung des Pulvers nach Anspruch 1, worin das Pulver Huminsäuresalze von +1-wertige entweder Na- oder K-Salz enthält.

4. Verwendung des Pulvers nach Anspruch 1, worin das Pulver Huminsäuresalze, von + 2-wertige entweder Ca-, Mg- oder Ba-Salz enthält.

5. Verwendung des Pulvers nach Anspruch 1, worin das Pulver Huminsäuresalze, von +3-wertige entweder Fe- oder Al-Salz enthält.

6. Verwendung des Pulvers nach Anspruch 1, worin das Pulver eine durchschnittliche Korngröße zwischen 10 und 20 µm hat.

## Revendications

1. Utilisation d'une poudre comprenant de l'acide humique ou des sels d'acide humique (humate) pour la détection d'empreintes digitales.

2. Utilisation de la poudre selon la revendication 1, dans laquelle ladite poudre comprend en outre environ 10 à 20% en poids de carbone noir.

3. Utilisation de la poudre selon la revendication 1, dans laquelle ladite poudre comprend les sels d'acide humique dans la valence +1 du sel de Na ou de K.

4. Utilisation de la poudre selon la revendication 1, dans laquelle ladite poudre comprend les sels d'acide humique dans la valence +2 de sel de Ca, Mg ou Ba.

5. Utilisation de la poudre selon la revendication 1, dans laquelle ladite poudre comprend les sels d'acide humique dans la valence + 3 de sel de Fe ou d'Al.

6. Utilisation de la poudre selon la revendication 1, dans laquelle ladite poudre a une taille de grain moyenne entre 10 et 20 µm.
